# EUROPEAN PATENT APPLICATION

(11) **EP 0 598 474 A1**
(43) Date of publication of application: **25.05.1994**
(21) Application number: 93306731.6
(22) Date of filing: 24.08.1993
(51) Int. Cl.: C08B 37/10, A61K 31/725

(54) **2-Deoxy-2-amino hexuronyl derivatives of heparin and a method for production**

(30) Priority: 25.08.1992 BE 9200751
(71) Applicant: SYNTEX S.A., Buenos Aires 1035 (AR)
(72) Inventor: Diaz, Victor Bautista, (1035) Buenos Aires (AR); Domanico, Ricardo Hugo, (1035) Buenos Aires (AR); Fussi, Fernando, CH-6900 Lugano (CH)
(74) Representative: Perry, Robert Edward

(57) **Abstract**

A heparin containing 2-deoxy-2-amino-L-iduronate units has therapeutic utility.

## Description

### FIELD OF THE INVENTION

Heparin a complex heteropolysaccharide obtained by extraction from specific animal tissues, contains repeating units of hexuronyl-glucosamine-N-sulfate. Hexuronyl residues in the disaccharide unit are reprepresented by D-glucuronate and L-iduronate. Other sulfate groups are located in position C-2 of L-Iduronate moiety and position 6 of glucosamine-N-sulfate moiety.

Therefore, Heparin can be described as a polymer of: 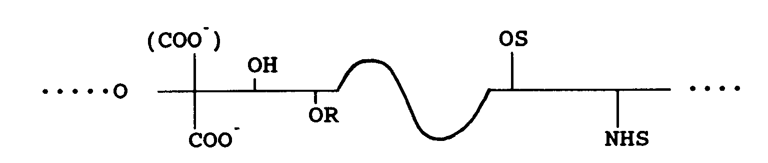
L-iduronylsulfate 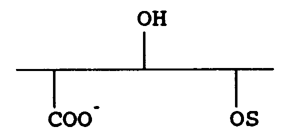
represents the main form in which uronyl residues are present in Heparin.

The products for which we apply for a patent are Heparin derivatives containing the follcwing disaccharide unit: 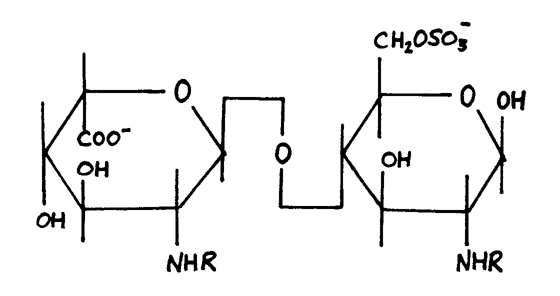
where R=H, SO₃- o CH₃CO

Henceforth, these structures and all the structures considered in this application will be described with the following abbreviated symbols:

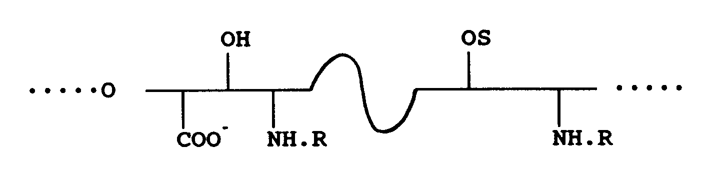

Heparin is a well known therapeutical agent endowed with activities related to topics as blood clotting, fibrinolysis, lipid clearing, cell growth. Unfortunately, heparin presents also some draw-back as : risk of hemorrhages, scarce bioavailability, short half-life, interference with platelet aggregation.

These considerations prompted many Laboratories to introduce some modifications into the structure of the original molecule, in order to minimize the unrequested properties, to modulate the multiple effects, to increase bioavailability and half-life.

### DESCRIPTION OF THE PRIOR ART

Although many chemical methods have been described to depolymerize heparin or to introduce substituents in the amino group of glucosamine, very little is known about possible substitution in positions 2 and 3 of iduronyl-2-sulfate.

We described how to enter acyl groups in position (US. Patent 5,011,919).

And in this patent we describe a method to obtain in one step, a new heparin derivative (I) having a free amino group in position 2 of the L-iduronil residue. The importance of the reaction leading to I resides in the fact that as far as we know, no other points on the molecule will be altered.

### PREFERRED EMBODIMENT

Compound I is an ideal starting material to obtain a new heparin analog (II) or mono- and di-acetylated compounds III and IV with reduced ionicity with respect to heparin and, therefore, a better bioavailability.

The new compounds I, II, III and IV will be described and characterized.

The scheme for overall reaction is as follows: 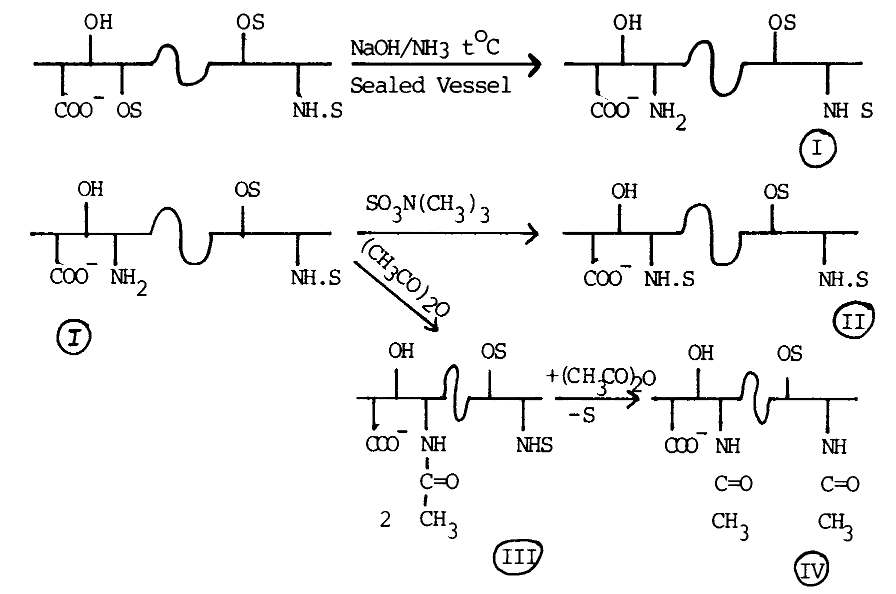
1) The reaction from Heparin leading to compound I is performed in a closed. pressure-proof vessel at 60 - 80^{º}C for 3-4 hs. More hours of reaction cause a progressive loss on anti-clotting activity but no significative structural modifications as shown by the constant molar ration N/S and by NMR spectrum. Heparin is dissolved (10%0) in concentrated (25% w/v) amonium hydroxide and 0.05 vol. of 10 M NaOH are added to obtain a final concentration of 0.5 M NaOH. The solution is kept in a sealed reactor plunged in a water bath at 70^{º}C for about 4 hours. Compound I is collected by repeated precipitations with ethanol.
2) Compound II is obtained from i with amine sulfotrioxide (pyridin sulfotrioxide, trimethylamine sulfotrioxide) in buffered solution at pH: 9.0. The excess sulfotrioxide is removed by filtration and compound II is recovered by saline (2M NaCl)/ethanol precipitation.
3) Compounds III and IV are obtained by treatment with acetic anhydride, pH: 9.0-9.5, de N-sulfation in methanol/sulfuric acid (0,5% w/v H2SO4), and further treatment with acetic anhydride. Compounds III and IV are recovered by ethanol precipitation.

Example 1 describes the preparation of Compound I. Examples 2 and 3 describe the further steps to Compound II and to Compound IV through Compound III.

Table 1 reports the main characters of I to IV and of the starting Heparin.

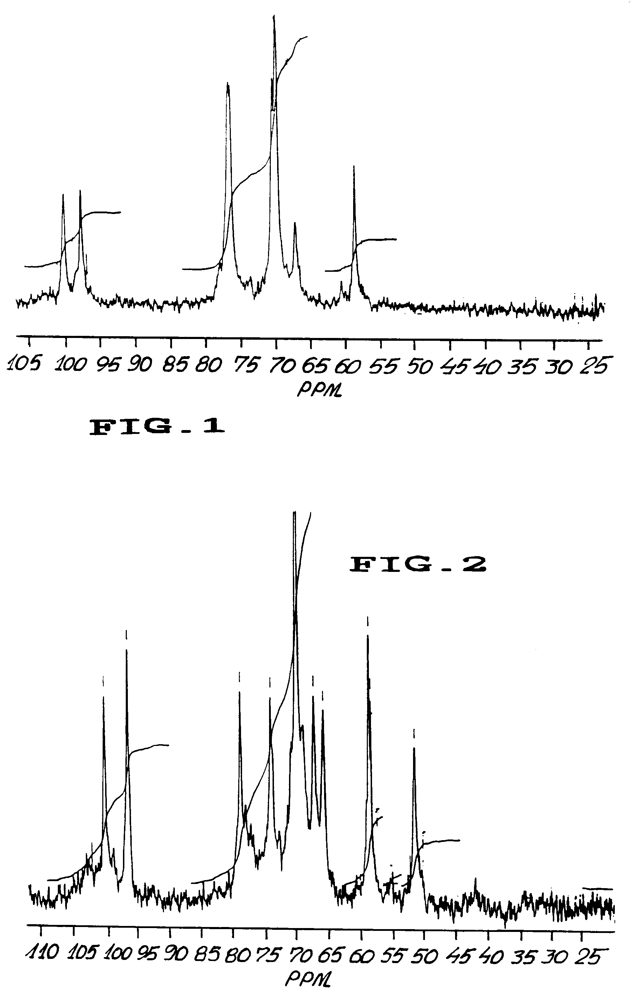

Fig. 1 and 2 present the 13C-NMR spectra, corresponding to Heparin and Compound I.

| | HEPARIN | COMPOUND I | COMPOUND II | COMPOUND III | COMPOUND IV |
|---|---|---|---|---|---|
| Total Nitrogen | 2,2 % | 3,7 % | 3,4 % | 3,6 % | 4,0 % |
| Total Sulphur | 12,2 % | 10,5 % | 12,1 % | 10,0 % | 5,2 % |
| Ratio S/N | 2,43 | 1,24 | 1,56 | 1,21 | 0,57 |
| USP Potency (IU/mg) | 150 | 60 | 100 | 55 | 2 |
| CH,CO⁻ Dosage (CGL) | abs. | abs. | abs. | 3,6 % | 12,0 % |

### Example N^{º} 1

10 g of injectable heparin, potency 150 ui/mg, are dissolved 10% w/v in a solution of concentrated ammonium hydroxide (25% w/v) that contains HONa in enough quantity for 0,5M.

It is heated in a sealed reactor for 4 hs at 70 C; once it finish, it is added 5g of NaCl and 300 ml of ethanol.

The precipitated obtained is dissolved in 100 ml of deionized water and is dialyzed in cellophane membrane versus a 0.5M NaCl solution (5 liters).

The dialyzed solution is precipitated adding 2 volumes of ethanol; the solid isolated is anhydrated with absolute ethanol and is dried in a vacuum, stove during 24 hs. at 60 C.

The yield is 6,5g (Compound I).

### Example N^{º} 2

4 g of compound I are dissolved in 40 ml of distillated water, adding 2 g of NaHCO3 and 2g of SO3N(CH3) under agitation. It is kept 12 hs, under these conditions; afterwards is filtrated to eliminate the residual sulfotrioxide, the pH is adjusted to 6 with HCl 1M; 5g of NaCl and 150 ml of ethanol are added.

The solid obtained is dehydrated with absolute ethanol and dried in vacuum stove, during 24 hs at 60 C.

The yield is 3,8g (Compound II).

### Example N^{º} 3

10 g of Compound I are dissolved in 100 ml of deionized water. Adjust pH to 9.0-9.5 with CO3Na2 solution (20% w/v); add 2 ml of acetic anhydride by dropping, keeping the pH controlled by adding CO3Na2 solution.

After 30 minutes of agitation. isolate the reaction product, adjusting previously the pH to 6 with HCl 1M by addition of 5g of NaCl and 200 ml of ethanol

The solid is dehydrated with absolute ethanol and is dried in vacuum stove during 24 hs at 60 C.

The yield is 9g (Compound III).

### Example N^{º} 4

10 g of Compound III are suspended in 100 ml of methanol that contains 0,5 g of concentrated sulfuric acid. It is heated under reflux during 60 minutes at 60^{º}C. The solid is filtrated and dissolved in 100 ml of deionized water: the pH is adjusted to 9.0-9.5 with a Na2CO3 solution (20% w/v), adding 2 ml of acetic anhydride by dropping and agitation, keeping the pH controlled by adding a solution of CO3Na2. After 30 minutes of agitation, the reaction product is isolated, adjusting the pH to 6 with HCl 1M, adding 5 g of NaCl and 200 ml of ethanol.

The solid obtained is dehydrated with absolute ethanol and dried in vacuum stove during 24 hs at 60 C.

The yield is 6 g of Compound IV.

## Claims

1. A heparin containing 2-deoxy-2-amino-L-iduronate units.

2. A heparin according to claim 1, obtainable by reacting heparin in a closed reactor, at 60-80°C for 4-5 hours, with 0.5 M sodium hydroxide in concentrated ammonium hydroxide (20-25% w/v).

3. A heparin according to claim 1 or claim 2, wherein the amino group is sulfated and/or acylated.

4. A heparin according to any preceding claim, for use in therapy.

5. Use of a heparin according to any of claims 1 to 3, for the manufacture of a medicament for use as a replacement for heparin.
